Europäisches Patentamt

European Patent Office

Office européen des brevets

(19)

(11) Veröffentlichungsnummer : **0 014 007**
**B1**

(12) # EUROPÄISCHE PATENTSCHRIFT

(45) Veröffentlichungstag der Patentschrift :
13.01.82

(21) Anmeldenummer : 80200022.4

(22) Anmeldetag : 10.01.80

(51) Int. Cl.³ : **C 12 M 3/04, C 12 M 1/20**

(54) **Biologisches Gefäss.**

(30) Priorität : 19.01.79 DE 2902026

(43) Veröffentlichungstag der Anmeldung :
06.08.80 (Patentblatt 80/16)

(45) Bekanntmachung des Hinweises auf die Patenterteilung : 13.01.82 Patentblatt 82/02

(84) Benannte Vertragsstaaten :
AT BE CH DE FR GB IT LU NL SE

(56) Entgegenhaltungen :
DE - A - 2 510 183
DE - B - 2 157 150
US - A - 3 726 767
US - A - 3 883 398

(73) Patentinhaber : **Peters, J. Hinrich, Dr.**
**Lüghauser Strasse 70**
**D-5064 Rösrath-Hoffnungsthal (DE)**

(72) Erfinder : **Peters, J. Hinrich, Dr.**
**Lüghauser Strasse 70**
**D-5064 Rösrath-Hoffnungsthal (DE)**

(74) Vertreter : **Eggert, Hans-Gunther, Dr.**
**Raederscheidtstrasse 1**
**D-5000 Köln 41 (DE)**

Imprimerie Jouve, 18, rue St-Denis, 75001 Paris, France

Die Erfindung betrifft ein biologisches Gefäß für Zellkulturen oder biologische Tests mit einem lösbaren und flüssigkeitsdicht mit den Seitenwänden des Gefäßes verbundenen Boden, wobei der Boden und die Seitenwände wenigstens eine Kammer umschließen, während die Seitenwände einen Wandkörper bilden, und ggf. mit einer Abdeckung für die Kammer(n).

Ein derartiges Gefäß ist aus der DE-B-2 157 150 bekannt, bei dem der Boden mit dem Wandkörper über eine klebfähige Dichtung verbunden ist, wobei die Dichtung auf dem Boden aufgeklebt ist und von Nuten des Wandkörpers aufgenommen wird. Der Boden dient hierbei als festes Substrat zum Anheften von lebenden Zellen höherer Organismen, die unter sterilen Bedingungen in geeigneten Kulturmedien in einer Zellkultur gezüchtet werden. In der Zellbiologie ist es in vielen Fällen erforderlich, die auf einer Oberfläche gewachsenen Zellen außerhalb der Kultur zu analysieren, sei es mit dem Mikroskop, mit biochemischen Methoden oder mit Verfahren zur Bestimmung der aufgenommenen Radioaktivität. Nach Beendigung der Kultur wird daher bei diesem Gefäß der Wandkörper vom Boden manuell gelöst. Die mit dem Boden verklebte Dichtung verbleibt jedoch zunächst und ist ebenfalls durch Abkratzen zu entfernen, was jedoch sehr mühsam ist. Aus diesem Grunde eignen sich für diesen Zweck auch praktisch nur Böden aus Glas, die jedoch nicht für alle Zelltypen das geeignete Substrat zum Anheften darstellen. Abgesehen davon ist ein derartiges Gefäß aufgrund der aufzuklebenden Dichtung aufwendig in der Herstellung und kann nur einmalig benutzt werden.

Aus der US-A-3 883 398 ist ein Gefäß für Mikrokulturen bekannt, bei dem eine mit durchgehenden Öffnungen versehene Matrix auf einem Boden aufliegt und gegen diesen abgedichtet ist und anderseitig durch eine mit korrospondierenden Öffnungen versehene Platte bedeckt ist, wobei die gesamte Einrichtung durch seitliche Klammerschienen zusammengehalten wird.

Aus der US-B-3 726 767 ist ein mikrobiologisches Reaktionsgefäß bekannt, das einen entlang eines tunnelförmigen Ansatzes verschiebbaren Boden aufweist und durch eine Haube abdeckbar ist.

Aufgabe der Erfindung ist es, ein Gefäß der eingangs genannten Art zu schaffen, das herstellungsmäßig wesentlich vereinfacht ist und bei dem das Ablösen und Weiterverwenden des Bodens mit den darauf befindlichen Zellkulturen ohne weiteres und ohne zusätzliche Maßnahmen möglich ist.

Diese Aufgabe wird dadurch gelöst, daß von dem Wandkörper wenigstens der am Boden angrenzende Teil aus einem am Boden durch Selbstadhäsion flüssigkeitsdicht haftenden, kautschukelastischen, nicht zytotoxischen Kunststoff besteht.

Dadurch, daß der Wandkörper mit dem Boden nur durch Adhäsionshaftung verbunden ist, können diese beiden Teile mühelos voneinander getrennt werden. Es entfällt ein Anbringen einer Dichtung, der Boden und der Wandkörper brauchen lediglich miteinander in Berührung gebracht und ggf. aneinander gedrückt zu werden, um die Adhäsionshaftung hervorzurufen. Abgesehen davon kann dann der Wandkörper als Ganzes wieder verwendet werden, in dem er nach Gebrauch beispielsweise in einer Lösung eines nicht toxischen Detergenz gewaschen, mit destilliertem Wasser gespült und anschließend durch Autoclavieren sterilisiert wird.

Der Wandkörper kann auch zweischichtig ausgebildet sein, wobei die dem Boden zugewandte Schicht aus dem am Boden durch Adhäsion flüssigkeitsdicht haftenden, kautschukelastichen, nicht zytotoxischen Kunststoff besteht. Entsprechendes gilt für die dem Boden abgewandte Fläche des Wandkörpers, jedoch kann der gesamte Wandkörper auch aus dem Kunststoff bestehen. Bei größerflächigen Gefäßen mit vielen Kammern kann der Wandkörper ferner eine in diesem eingebettete Verstärkungsplatte aus praktisch starrem Material enthalten.

Die Haftfläche des Wandkörpers kann eben und glatt sein, jedoch sind auch konkav gewölbte Haftflächen möglich. Eine Haftung ist ebenfalls möglich, wenn die Haftfläche des Wandkörpers anstatt spiegelglatt zu sein, seidenmatt, d.h. im Mikromaßstab rauh ist. Weiterhin kann die Haftfläche durch rillenartige Vertiefungen unterbrochen sein, um geringe Unterschiede im thermischen Ausdehnur verhalten der beiden aneinanderhaftenden Materialien abzufangen.

Der Wandkörper besteht vorzugsweise aus einem hydrophoben Kunststoff und insbesondere aus Silikonkautschuk.

Andere für die Zwecke der Erfindung geeignete Kunststoffe sind beispielsweise :

1. Polyvinylchlorid mit Zusatz von hochmolekularen Weichmachern, besonders Äthylen-Vinylacetat-Copolymerisat. Der Vinylacetat-Gehalt kann dabei bis zu 90 % betragen, was zu besonders weichen Verbindungen führt. Durch Variation des Äthylen-Anteils kann der Grad der Hydrophobie variiert werden.

2. Polyurethan-Elastomere mit unterschiedlichen Alkohol-Anteilen.

3. Polyvinyliden-Chlorid.

4. Methyl-Kautschuk bzw. Chlorid-Kautschuk, beide sind außerdem hydrophob, wobei die Eigenschaften dieser Substanzen durch Füllstoff- und Stabilisator-Zugabe variiert werden können, außerdem ist ein Zusatz von Siliconöl oder anderen Gleitmitteln möglich.

5. Fluorkautschuk, nicht perfluoriert, sondern mit Chlor kombiniert ergibt ebenfalls kautschukelastische, hydrophobe Verbindungen.

6. Polyurethan-Gele.

Der Boden kann ein Glasboden sein, zumal auch Glas an Silikonkautschuk haftet, jedoch wird ein Kunststoffboden bevorzugt, dessen Material eine entsprechende Haftfähigkeit an dem Wandkörper aufweist, zugleich aber auch einen für Zellkulturen besonders geeigneten Boden darstellt. Beispielsweise eignen sich hierfür verbiegbare Platten, insbesondere von einer Stärke in der Größenordnung von etwa 0,5 mm etwa aus Polyurethan, Polystyrol oder dergleichen. Dieses stellt ein zellfreundliches Plastikmaterial dar, das einen für die Zellkulturen geeigneten Boden bildet.

Stattdessen kann aber auch ein Filterboden, etwa aus Polycarbonat verwendet werden, dessen Poren den Durchtritt von Molekülen, Partikeln oder Zellfortsätzen gestatten.

Die Kammern können einen lichten Querschnitt aufweisen, der sich zum Boden hin verjüngt, während die Höhe des Wandkörpers bzw. der Kammern 0,1 bis 20, insbesondere 2 bis 10 mm betragen kann.

Ferner kann das Gefäß mit einem locker sitzenden Deckel, insbesondere mit überfallenden Rand, versehen sein, wobei der Deckel aber ebenfalls mit Adhäsionshaftung an dem Wandkörper haften kann.

Die Erfindung wird nachfolgend anhand der in den beigefügten Abbildungen dargestellten Ausführungsbeispiele näher erläutert, in denen ferner Maßangaben für verschiedene Ausführungsformen (in mm) angebracht sind.

Figuren 1a, 2a und 3a zeigen drei verschiedene Ausführungsformen eines erfindungsgemäßen biologischen Gefäßes perspektivisch.

Figuren 1b, 2b und 3b zeigen die Ausführungsformen der Figuren 1a, 2a und 3a im Schnitt.

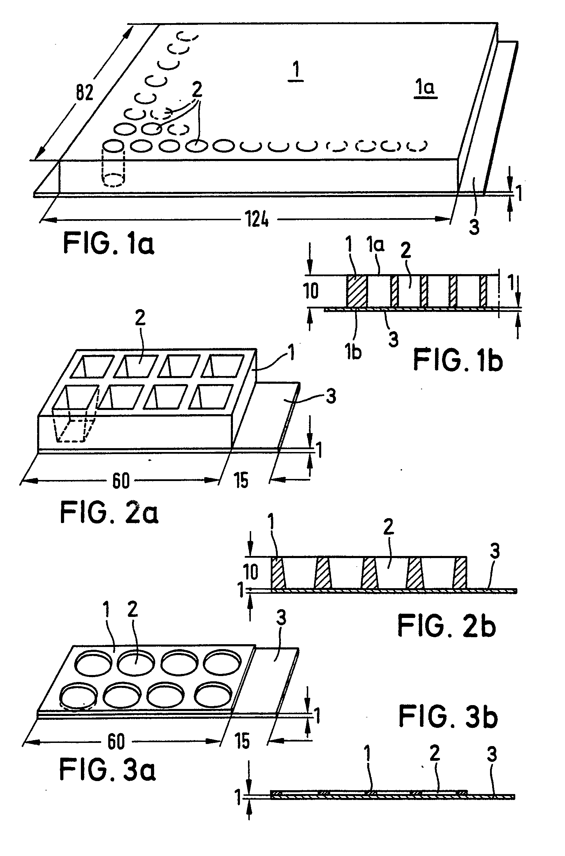

Das in den Figuren 1a und 1b dargestellte biologische Gefäß besteht aus einem Wandkörper 1 mit einer relativ großen Fläche und einer großen Vielzahl an zylindrischen Bohrungen, die sich in einer gewünschten Anordnung von der Oberseite 1a zur Unterseite 1b des Wandkörpers 1 erstrecken und an beiden Seiten offen sind. Der Wandkörper 1 haftet auf einem Boden 3, so daß die Bohrungen an ihrer Unterseite durch den Boden 3 flüssigkeitsdicht verschloßen werden und auf diese Weise eine Vielzahl von Kammern 2 gebildet wird. Der Boden 3 steht hierbei an zwei gegenüberliegenden Seiten gegenüber dem Wandkörper 1 über.

Bei der in den Figuren 2a und 2b dargestellten Ausführungsform bestehen die Kammern 2 aus quadratischen, sich zum Boden 3 hin verjüngenden Durchtrittsöffnungen im Wandkörper 1.

Bei der in den Figuren 3a und 3b dargestellten Ausführungsform sind der Wandkörper 1 und damit auch die Kammern 2 sehr flach ausgebildet.

Die Böden 3 sind insbesondere flexibel und können aus Polystyrol, Polyäthylen, Polypropylen, Polycarbonat und fluorierten polymerisierten Kohlenwasserstoffen bestehen. Jedoch lassen sich auch mikroskopische Objektträger aus Glas verwenden.

Andererseits besteht auch die Möglichkeit, Dialyse- und Filterfolien als Boden 3 zu benutzen, die je nach Porengröße den Durchtritt von Molekülen, Partikeln oder Zellfortsätzen gestatten. Zu diesem Zweck wird dann das Gefäß in eine entsprechende Flüssigkeit gestellt, die durch die Poren mit dem Inneren der Kammern 2 kommunizieren kann.

Die Böden 3 können, bevor sie mit dem Wandkörper 1 zusammengebracht werden, bereits Testsubstanzen aufweisen, z.B. Testreagenzien, Antiseren, Viruspreparationen und dergleichen. Diese Substanzen läßt man in dem Raster der späteren Kammern 2 auf den Feldern der Böden 3 auftrocknen. Hierdurch wird das standardisierte Testen von Reagenzien mit ihrer Wirkung auf Zellen oder andere biologische Präparationen erleichtert, was im übrigen kostengünstig ist, da Lagern, Verpacken und Verwenden derartiger Böden 3 preiswerter und einfacher ist als der Gebrauch von ganzen entsprechend präparierten Zellkulturgefäßen.

Zum Gebrauch werden Wandkörper 1 und Boden 3 zusammengebracht, zu welchem Zweck ggf. ein Rahmen verwendet werden kann, um die genaue Ausrichtung von Wandkörper 1 und Boden 3 zu erreichen. Dann werden die Kammern 2 mit der Suspension von zu kultivierenden Zellen gefüllt. An den Zellen werden experimentelle Manipulationen entsprechend dem Vorgehen bei normalen Kulturen vorgenommen. Die Kammern 2 können entweder in eine sterile Petrischale gestellt und mit aufgesetztem Petrischalendeckel unter Standardbedingungen kultiviert oder mit einem eigenen Deckel versehen werden. Der Deckel kann dabei entweder locker aufsitzen und Belüftungsnocken und einen überfallenden Rand aufweisen, oder er kann an dem Wandkörper 1 durch Adhäsion haften und somit die Kammer 2 wie auf der Unterseite durch den Boden 3 abschließen. In diesem Falle kann die Kammer 2 als geschlossenes System kultiviert werden. Für biologische Tests kann das Gefäß zum Testen nichtzellulärer Reaktionen, z.B. Agglutinationsreaktionen mit Latexkugeln oder mit abgetöteten Zellen, verwendet werden.

Zum Zeitpunkt der Beendigung der Kultur kann der Boden 3 ohne weiteres von dem Wandkörper 1 getrennt werden, wobei die Zellen vor oder nach dem Trennen fixiert werden können. Sollen nicht anhaftende Zellen bzw. Produkte von Farb-oder Agglutinationsreaktionen auf dem Boden 3 erhalten und archiviert werden, so sind sie vorher mit einem geeignetem Fixierungsmittel, z.B. einer konzentrierten Proteinlösung, aufzutrocknen.

Nicht fixierte, angeheftete Zellen können jedoch auch nach dem Abtrennen des Wandkörpers 1 auf dem Boden 3 weiter kultiviert werden, indem der Boden 3 in eine Kulturschale mit Kulturmedium gelegt wird.

Der Boden 3 mit der Zellkultur kann einen mikroskopischen Objektträger bilden oder zusätzlich auf einen Glasobjektträger aufgebracht werden. Eine Auswertung mit Hilfe histologischer Färbung, histochemischen Reaktionen oder Immunfluoresenz ist möglich.

Nach vorheriger Markierung der kultivierten Zellen kann eine mikroskopische Autoradiographie vorgenommen werden, bei der sich fotographische Silberkörner über dem Ort des Einbaus der radioaktiven Substanz bilden.

Radioaktiv markierte Zellen auf den Böden 3, insbesondere auf toluolunempfindlichen transparenten Materialien, können auf ihre Radioaktivität durch Szintillationsmessung untersucht werden. Besonders geeignet hierfür sind Plastikplatten, die in die einzelnen Kulturareale zerschnitten werden können, die getrennt gemessen werden.

Auf Plastikplatten als Böden 3 können ferner Zellen für die Elektronenmikroskopie eingebettet und ultradünn geschnitten werden.

Eine Ausführungsform des Gefäßes nach den Figuren 1a und 1b eignet sich besonders zur Handhabung des Mikrotitersystems, für das eine Vielzahl von Zubehörgeräten existiert. Diese Geräte erleichtern das Pipetieren von großen Serien ebenso wie das Absammeln von Kulturüberständen oder suspendierten Zellen zu ihrer weiteren Auswertung.

Zwecks Untersuchung mit einem Szintillationszähler können Böden 3 aus toluolbeständigem Kunststoffmaterial sowohl mit der Schere zerschnitten als auch mit einer Stanze in entsprechende Felder zerteilt werden, die dann direkt in ein darunter stehendes Gläschen mit Szintillatorflüssigkeit fallen.

Nach Beendigung der vorzunehmenden Untersuchungen kann der unterzeilte trockene Boden 3 mit der darauf befindlichen Zellkultur etwa in Folien- oder Plattenform kompakt archiviert werden.

Ferner ist es möglich, die Zellen in den einzelnen Kammern 2 zu kultivieren und radioaktiv zu markieren, wonach dann nach Beendigung der Kultur der Boden 3 an dem Wandkörper 1 belassen und zusätzlich ein weiterer Wandkörper 1 von der anderen Seite des Bodens 3 paßgerecht gegen diesen gesetzt wird, wobei der Boden 3 als Filterfolie ausgebildet ist. Diese Kombination bestehend aus zwei Wandkörpern 1 und einem Boden 3 kann dann von oben mit Waschflüssigkeit beschickt werden, die gleichzeitig nach unten abgesaugt wird. Durch die Wahl der Waschflüssigkeiten werden die an sich suspendierten Zellen auf dem als Filter ausgebildeten Boden 3 festgehalten aufgeschlossen und je nach Wunsch extrahiert, so daß schließlich das unlösliche Material auf dem Boden 3 zurückbleibt. Danach kann der Boden 3 entnommen und die einzelnen Proben einer Szintillationsmessung unterworfen werden.

**Ansprüche**

1. Biologisches Gefäß für Zellkulturen oder biologische Tests mit einem lösbaren und flüssigkeitsdicht mit den Seitenwänden des Gefäßes verbundenen Boden, wobei der Boden und die Seitenwände wenigstens eine Kammer umschließen, während die Seitenwände einen Wandkörper bilden, und ggf. mit einer Abdeckung für die Kammer(n), dadurch gekennzeichnet, daß von dem Wandkörper (1) wenigstens der an den Boden (3) angrenzende Teil aus einem am Boden (3) durch Selbstadhäsion flüssigkeitsdicht haftenden, kautschukelastischen, nicht zytotoxischen Kunststoff besteht.

2. Gefäß nach Anspruch 1, dadurch gekennzeichnet, daß die dem Boden (3) abgewandte Fläche des Wandkörpers (1) ebenfalls aus dem Kunststoff besteht.

3. Gefäß nach einem der Ansprüche 1 oder 2, dadurch gekennzeichnet, daß der ganze Wandkörper (1) aus dem Kunststoff besteht.

4. Gefäß nach einem der Ansprüche 1 bis 3, dadurch gekennzeichnet, daß die Haftfläche des Wandkörpers (1) eben und glatt ist.

5. Gefäß nach einem der Ansprüche 1 bis 4, dadurch gekennzeichnet, daß der Wandkörper (1) aus einem hydrophoben Kunststoff besteht.

6. Gefäß nach einem der Ansprüche 1 bis 5, dadurch gekennzeichnet, daß der Kunststoff ein Silikonkautschuk ist.

7. Gefäß nach einem der Ansprüche 1 bis 6, dadurch gekennzeichnet, daß sich der lichte Querschnitt der Kammer(n) (2) zum Boden (3) verjüngt.

8. Gefäß nach einem der Ansprüche 1 bis 7, dadurch gekennzeichnet, daß die Höhe des Wandkörpers (1) bzw. der Kammern(n) (2) 0, 1 bis 20, insbesondere 2 bis 10 mm, beträgt.

9. Gefäß nach einem der Ansprüche 1 bis 8, gekennzeichnet durch einen Boden (3) aus zellfreundlichem Plastikmaterial.

10. Gefäß nach einem der Ansprüche 1 bis 9, gekennzeichnet durch einen Filterboden (3), dessen Poren den Durchtritt von Molekülen, Partikeln oder Zellfortsätzen gestatten.

11. Gefäß nach einem der Ansprüche 1 bis 10, gekennzeichnet durch einen locket sitzenden Deckel, insbesondere mit überfallendem Rand.

12. Gefäß nach einem der Ansprüche 1 bis 10, gekennzeichnet durch einen adhärenten Deckel.

13. Gefäß nach einem der Ansprüche 1 bis 12, dadurch gekennzeichnet, daß der Wandkörper (1) eine Haftfläche aufweist, die seidenmatt und/oder durch rillen- oder napfförmige Vertiefungen unterbrochen ist.

14. Gefäß nach einem der Ansprüche 1 bis 13, dadurch gekennzeichnet, daß auf dem Boden (3) Testsubstanzen entsprechend dem Raster der Kammern (2) des Wandkörpers (1) aufgebracht sind.

15. Gefäß nach einem der Ansprüche 1 bis 14, dadurch gekennzeichnet, daß ein Rahmen zum deckungsgleichen Ineinandersetzen von Wandkörper (1) und Boden (3) vorgesehen ist.

**Claims**

1. Biological container for cell cultures or biological tests having a base which is detachable and connected in a fluid-tight manner with the side walls of the container, in which the base and the side walls enclose at least one compartment, whilst the side walls form a wall body, and optionally having a cover for the compartment(s), characterised in that at least that part of the wall body (1) which adjoins the base (3) consists of a rubber elastic, non-cytotoxic synthetic material, which adheres in a fluid-tight manner to the base (3) by self-adhesion.

2. Container according to Claim 1, characterised in that the surface of the wall body (1) remote from the base (3) also consists of the synthetic material.

3. Container according to one of Claims 1 or 2, characterised in that the entire wall body (1) consists of the synthetic material.

4. Container according to one of Claims 1 to 3, characterised in that adhesion surface of the wall body (1) is even and smooth.

5. Container according to one of Claims 1 to 4, characterised in that the wall body (1) consists of a hydrophobic synthetic material.

6. Container according to one of Claims 1 to 5, characterised in that the synthetic material is a silicone rubber.

7. Container according to one of Claims 1 to 6, characterised in that the interior cross-section of the compartment(s) (2) tapers towards the base (3).

8. Container according to one of Claims 1 to 7, characterised in that the height of the wall body (1) or compartment(s) (2), is 0.1 to 20 mm, particularly 2 to 10 mm.

9. Container according to one of Claims 1 to 8, characterised by a base (3) of cell-compatible plastics material.

10. Container according to one of Claims 1 to 9, characterised by a filter base (3), the pores of which permit the passage of molecules, particles or cell growths.

11. Container according to one of Claims 1 to 10, characterised by a loosely fitting cover, particularly with an overlapping rim.

12. Container according to one of Claims 1 to 10, characterised by an adherent cover.

13. Container according to one of Claims 1 to 12, characterised in that the wall body (1) has an adhesion surface which has a matt-silk finish and/or is interrupted by groove- or cup-shaped depressions.

14. Container according to one of Claims 1 to 13, characterised in that test substances are applied on the base (3) according to the grid pattern of the compartments (2) of the wall body (1).

15. Container according to one of Claims 1 to 14, characterised in that a frame is provided for congruent fitting into one another of the wall body (1) and the base (3).

**Revendications**

1. Récipient biologique pour cultures de cellules ou tests biologiques, comportant un fond amovible, relié aux parois latérales du récipient avec étanchéité aux liquides et formant avec elles une chambre au moins, tandis que les parois latérales forment une plaque alvéolaire, ainsi que le cas échéant un couvercle pour la ou les chambres, ledit récipient étant caractérisé en ce qu'au moins la partie de la plaque alvéolaire (1) adjacente au fond (3) est constituée par une matière plastique non cytotoxique, présentant une élasticité caoutchoutique et se fixant sur le fond (3) par auto-adhérence avec étanchéité aux liquides.

2. Récipient selon revendication 1, caractérisé en ce que la surface de la plaque alvéolaire (1) opposée au fond (3) est également constituée par la matière plastique.

3. Récipient selon une des revendications 1 et 2, caractérisé en ce que l'ensemble de la plaque alvéolaire (1) est constitué par la matière plastique.

4. Récipient selon une quelconque des revendications 1 à 3, caractérisé en ce que la surface adhérente de la plaque alvéolaire (1) est plane et lisse.

5. Récipient selon une quelconque des revendications 1 à 4, caractérisé en ce que la plaque alvéolaire (1) est constituée par une matière plastique hydrophobe.

6. Récipient selon une quelconque des revendications 1 à 5, caractérisé en ce que la matière plastique est un caoutchouc silicone.

7. Récipient selon une quelconque des revendications 1 à 6, caractérisé en ce que la section utile de la ou des chambres (2) diminue vers le fond (3).

8. Récipient selon une quelconque des revendications 1 à 7, caractérisé en ce que la hauteur de la plaque alvéolaire (1) ou de la ou des chambres (2) est comprise entre 0,1 et 20 mm, et notamment entre 2 et 10 mm.

9. Récipient selon une quelconque des revendications 1 à 8, caractérisé par un fond (3) en matière plastique favorable à la culture de cellules.

10. Récipient selon une quelconque des revendications 1 à 9, caractérisé par un fond filtrant (3), dont les pores permettent le passage de molécules, particules ou ramifications cellulaires.

11. Récipient selon une quelconque des revendications 1 à 10, caractérisé par un couvercle simplement posé, comportant en particulier un bord en surplomb.

12. Récipient selon une quelconque des revendications 1 à 10, caractérisé par un couvercle adhérant.

13. Récipient selon une quelconque des revendications 1 à 12, caractérisé en ce que la plaque alvéolaire (1) présente une surface adhérente soyeuse et lisse et/ou interrompue par des cavités sous forme de rayures ou de godets.

14. Récipient selon une quelconque des revendications 1 à 13, caractérisé par le dépôt sur le fond (3) de substances de test suivant le quadrillage des chambres (2) de la plaque alvéolaire (1).

15. Récipient selon une quelconque des revendications 1 à 14, caractérisé par un cadre pour l'assemblage en recouvrement de la plaque alvéolaire (1) et du fond (3).

FIG. 1a

FIG. 1b

FIG. 2a

FIG. 2b

FIG. 3a

FIG. 3b